# EUROPEAN PATENT SPECIFICATION

(11) **EP 1 683 525 B1**
(45) Date of publication and mention of the grant of the patent: **17.05.2017**
(21) Application number: 04818538.3
(22) Date of filing: 15.11.2004
(51) Int. Cl.: A61K 31/517, A61K 9/16, A61K 9/20, A61K 9/22, A61K 9/28, A61K 31/519, A61K 31/53, A61K 31/549, A61K 47/14, A61K 47/32, A61K 47/36, A61K 47/38, A61K 47/42, A61K 47/44, A61P 1/00, A61P 29/00

(54) **SUSTAINED-RELEASE PHENYLALANINE DERIVATIVE PREPARATION FOR ORAL ADMINISTRATION**
PHENYLALANIN-DERIVAT-PRÄPARAT MIT VERZÖGERTER FREISETZUNG ZUR ORALEN VERABREICHUNG
PREPARATION DE DERIVES DE PHENYLALANINE A LIBERATION SOUTENUE POUR UNE ADMINISTRATION ORALE

(30) Priority: 14.11.2003 JP 2003385502
(43) Date of publication of application: 26.07.2006
(60) Divisional application: 17166067.3
(73) Proprietor: EA Pharma Co., Ltd., Tokyo 104-0042 (JP)
(72) Inventor: OGAWA, Kenichi, c/o AJINOMOTO CO., INC., Kawasaki-shi, Kanagawa 2108681 (JP); HAGIO, Hirokazu, c/o AJINOMOTO CO., INC., Kawasaki-shi, Kanagawa 2108681 (JP); HIGUCHI, Hiroyuki, c/o AJINOMOTO CO., INC., Kawasaki-shi, Kanagawa 2108681 (JP); ISHIKAWA, Motoki, c/o AJINOMOTO CO., INC., Kawasaki-shi, Kanagawa 2108681 (JP); YABUKI, Akira, c/o AJINOMOTO CO., INC., Kawasaki-shi, Kanagawa 2108681 (JP)
(74) Representative: Nicholls, Kathryn Margaret
(86) International application number: PCT/JP2004/016943
(87) International publication number: WO 2005/046697

(56) References cited:
- EP-A1- 1 288 205
- EP-A2- 0 988 863
- WO-A1-99/06437
- WO-A1-03/024426
- WO-A1-03/070709
- JP-A- 2002 534 439
- JP-A- 2003 238 507
- JP-B2- 3 440 469

## Description

### Technical Field of the Invention

The present invention relates to sustained-release oral administration preparations of phenylalanine derivatives or pharmaceutically acceptable salts thereof, which have an α 4 integrin inhibiting activity and are useful as agents for treating conditions such as inflammatory bowel disease.

### Background of the Invention

A compound of a formula (1) or pharmaceutically acceptable salts thereof have an α 4 integrin inhibiting activity and are useful compounds as agents for treating conditions such as inflammatory bowel disease. They can be produced in accordance with the description in Patent Literature 1. Though there is a description in this patent publication regarding ordinary tablets, granules, dispersants, pills, capsules, and sugarcoated agents, wherein the compound of the formula (1) or pharmaceutically acceptable salts thereof are dispensed, there is no disclosure of sustained-release oral administration preparations therein. Since the compound of the formula (1) or pharmaceutically acceptable salts thereof have a relatively shorter half-life period from blood plasma, QOL (quality of life) or compliance of patients may be improved by reducing doses of the above drugs.
[Patent Literature 1] WO02/16329 (EP-A-1 288 205)

In this context the document EP0988863 is relevant.

### Disclosure of the Invention

The object of the present invention is to provide a sustained-release oral administration preparation which allows the compound of the formula (1) or pharmaceutically acceptable salts thereof to exist longer in blood plasma.

The inventors have variously studied the above problem to solve it from the pharmaceutical point of view, and found that medicinal agents can become sustained-release by treating the compound of the formula (1) or pharmaceutically acceptable salts thereof with a water-soluble coating material(s) or a water-insoluble coating material(s) to prepare coated preparations. The present invention has been completed based on this finding.

Namely, the present invention relates to a sustained-release oral administration preparation having a core part containing an active ingredient, and a coating material for controlling release of the active ingredient from the core part, wherein the coating material is a water-soluble or a water-insoluble coating material, and the active ingredient is a phenylalanine compound of the following formula (1) or a pharmaceutically acceptable salt thereof, wherein A represents the following formula (2): 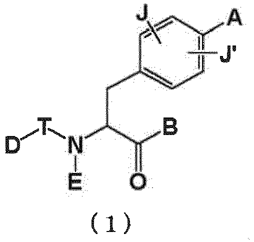 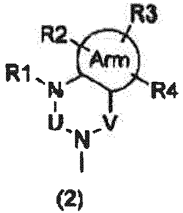
wherein Arm is a cyclic alkyl group or an aromatic ring having 0, 1, 2, 3, or 4 hetero atoms selected from the group consisting of an oxygen atom, sulfur atom and nitrogen atom; U and V represent C(=O), S(=O)₂ , C(-R5)(-R6), C(=C(R5)(R6)), C(=S), S(=O), P(=O)(-OH), or P(-H)(=O);
wherein R1, R2, R3, R4, R5, and R6 may be same or different from each other and represent a hydrogen atom, halogen atom, hydroxyl group, C₁₋₆ alkyl group, substituted C₁₋₆ alkyl group, C₂₋₆ alkenyl group, substituted C₂₋₆ alkenyl group, C₂₋₈ alkynyl group, substituted C₂₋₈ alkynyl group, cycloalkyl group which may have a hetero atom(s) in the ring, aryl group, heteroaryl group, C₁₋₆ alkoxy group, C₁₋₆ alkylthio group, C₁₋₆ alkoxy group and C₁₋₆ alkylthio group substituted with a cycloalkyl group(s) which may have a hetero atom(s) in the ring, C₁₋₆ alkoxy group and C₁₋₆ alkylthio group substituted with an aryl group(s), C₁₋₆ alkoxy group and C₁₋₆ alkylthio group substituted with a heteroaryl group(s), cycloalkyloxy group which may have a hetero atom(s) in the ring, aryloxy group, heteroaryloxy group, hydroxy C₁₋₆ alkyl group, hydroxy C₂₋₆ alkenyl group, hydroxy C₁₋₆ alkoxy group, halogeno C₁₋₆ alkyl group, halogeno C₁₋₆ alkoxy group, halogeno C₁₋₆ alkylthio group, halogeno C₂₋₆ alkenyl group, nitro group, cyano group, substituted or unsubstituted amino group, carboxyl group, C₁₋₆ alkyloxy carbonyl group, substituted or unsubstituted carbamoyl group, C₁₋₆ alkanoyl group, aroyl group, C₁₋₆ alkylsulfonyl group, substituted or unsubstituted sulfamoyl group, or ammonium group; R5 and R6 may bond together to form a ring, and in the ring, one or two oxygen atoms, nitrogen atoms or sulfur atoms may be included if necessary;
B represents a hydroxyl group, C₁₋₆ alkoxy group, or hydroxylamino group; E represents a hydrogen atom, C₁₋₆ alkyl group, C₂₋₆ alkenyl group, C₂₋₈ alkynyl group, C₁₋₆ alkyl group substituted with a cycloalkyl group(s) which may have a hetero atom(s) in the ring, C₁₋₆ alkyl group substituted with an aryl group(s), or C₁₋₆ alkyl group substituted with a heteroaryl group(s);
D represents a C₁₋₆ alkyl group, C₂₋₆ alkenyl group, C₂₋₈ alkynyl group, cycloalkyl group which may have a hetero atom(s) in the ring, aryl group, heteroaryl group, C₁₋₆ alkyl group substituted with a cycloalkyl group(s) which may have a hetero atom(s) in the ring, C₁₋₆ alkyl group substituted with an aryl group(s), or C₁₋₆ alkyl group substituted with a heteroaryl group(s), C₁₋₆ alkoxy group, C₁₋₆ alkoxy group substituted with a cycloalkyl group(s) which may have a hetero atom(s) in the ring, C₁₋₆ alkoxy group substituted with an aryl group(s), C₁₋₆ alkoxy group substituted with a heteroaryl group(s), a cycloalkyloxy group which may have a hetero atom(s) in the ring, aryloxy group, heteroaryloxy group, hydroxy C₁₋₆ alkyl group, hydroxy C₂₋₆ alkenyl group, hydroxy C₁₋₆ alkoxy group, halogeno C₁₋₆ alkyl group, halogeno C₁₋₆ alkoxy group, halogeno C₂₋₆ alkenyl group, nitro group, cyano group, substituted or unsubstituted amino group, carboxyl group, C₁₋₆ alkyloxy carbonyl group, substituted or unsubstituted carbamoyl group, C₁₋₈ alkanoyl group, aroyl group, C₁₋₆ alkylthio group, C₁₋₆ alkylsulfonyl group, or substituted or unsubstituted sulfamoyl group; E and D may bond together to form a ring, and in the ring, one or two oxygen atoms, nitrogen atoms or sulfur atoms may be included if necessary;
T represents an interatomic bond, C(=O), C(=S), S(=O), S(=O)₂, N(H)-C(=O), or N(H)-C(=S); and
J and J' may be same or different from each other and represents a hydrogen atom, halogen atom, C₁₋₆ alkyl group, C₁₋₆ alkyloxy group, or nitro group.

### Brief Description of the Drawings

Fig. 1 shows results of the dissolution test on Test Example 1. The horizontal axis indicates each example, and the vertical axis indicates the dissolution rate (wt%) of the compound (A) from a preparation two hours later.
Fig. 2 shows results of the dissolution test on Test Example 2. The horizontal axis indicates time (minutes), and the vertical axis indicates the dissolution rate (wt%) of the compound (A) from a preparation.
Fig. 3 shows results of the dissolution test on Test Example 2. The horizontal axis indicates time (minutes), and the vertical axis indicates the dissolution rate (wt%) of the compound (A) from a preparation.
Fig. 4 shows results of the dissolution test on Test Example 3. The horizontal axis indicates time (minutes), and the vertical axis indicates the dissolution rate (wt%) of the compound (A) from a preparation.

### Best Mode for Carrying out the Invention

In definitions of each group in the formulae (1) and (2) in the present specification, an alkyl group is a group having 1 to 6 carbon atoms and preferably having 1 to 4 carbon atoms. Alkyl groups, alkenyl group, and alkynyl groups as constituents of an alkyl group, alkenyl group, alkynyl group, alkoxy group, alkylthio group, alkanoyl group and alkylamino group can be linear or branched chains. The examples of alkyl groups include a methyl group, ethyl group, propyl group, isopropyl group, butyl group, sec-butyl group, tert-butyl group, pentyl group, and hexyl group, having 1 to 6 carbon atoms and preferably having 1 to 4 carbon atoms. The examples of alkenyl groups include a vinyl group, propenyl group, butenyl group, and pentenyl group, having 2 to 6 carbon atoms and preferably 2 to 4 carbon atoms. The examples of alkynyl groups include an ethynyl group, propynyl group, and butynyl group, having 2 to 8 carbon atoms and preferably 2 to 4 carbon atoms. A cycloalkyl group indicates a substituted or unsubstituted cycloalkyl group. The examples thereof include a cyclopropyl group, cyclobutyl group, cyclopentyl group, cyclohexyl group, norbornyl group, adamantyl group, and cyclohexenyl group, preferably having 3 to 8 carbon atoms and more preferably 3 to 5 carbon atoms. The examples of alkoxy groups include a methoxy group, ethoxy group, propyloxy group, and isopropyloxy group, having 1 to 6 carbon atoms and preferably 1 to 4 carbon atoms.

Hetero atoms include nitrogen, oxygen and sulfur. Halogen atoms represent fluorine, chlorine, bromine and iodine. The examples of halogeno alkyl groups include a chloromethyl group, trichloromethyl group, trifluoromethyl group, trifluoroethyl group, and pentafluoromethyl group. The examples of halogeno alkoxy groups include a trichloromethoxy group and trifluoromethoxy group. The examples of hydroxyalkyl groups include a hydroxymethyl group and hydroxyethyl group. A cycloalkyl group which may have a hetero atom(s) in the ring may be substituted or unsubstituted, and the examples thereof include preferably 4- to 8-membered ring and more preferably 5- to 7-membered ring of a cyclopentyl group, cyclohexyl group, piperidyl group, piperazinyl group, morpholinyl group, pyrrolidinyl group, tetrahydrofuranyl group, and uracil group.

An aryl group indicates a substituted or unsubstituted aryl group and includes, for example, a phenyl group, 1-naphthyl group, and 2- naphthyl group. A phenyl group and a substituted phenyl group are preferable among them, and a halogen atom, alkoxy group, alkyl group, hydroxyl group, halogeno alkyl group, and halogeno alkoxy group are particularly preferable as a substituent(s). A heteroaryl group indicates a substituted or unsubstituted heteroaryl group and includes, for example, a pyridyl group, pyrazyl group, pyrimidyl group, pyrazolyl group, pyrrolyl group, triazyl group, furyl group, thienyl group, isoxazolyl group, isothiazolyl group, indolyl group, quinolyl group, isoquinolyl group, benzoimidazolyl group, and imidazolyl group. A pyridyl group, pyrazyl group, pyrimidyl group, furyl group, thienyl group, imidazolyl group and substituted pyridyl, furyl, thienyl groups are preferable among them. A halogen atom, alkoxy group, alkyl group, hydroxyl group, halogeno alkyl group, and halogeno alkoxy group are particularly preferable as a substituent(s). A C₁₋₆ alkyl group substituted with an aryl group(s) includes a substituted or unsubstituted benzyl group, and a substituted or unsubstituted phenethyl group, for example. A halogen atom, alkoxy group, alkyl group, hydroxyl group, halogeno alkyl group, and halogeno alkoxy group are particularly preferable as a substituent(s). A C₁₋₆ alkyl group substituted with a heteroaryl group(s) includes a pyridylmethyl group, for example. A halogen atom, alkoxy group, alkyl group, hydroxyl group, halogeno alkyl group, and halogeno alkoxy group are particularly preferable as a substituent(s).

Examples of alkanoyl groups include a formyl group, acetyl group, propanoyl group, butanoyl group, and pivaloyl group. Examples of aroyl groups include a substituted or unsubstituted benzoyl and pyridylcarbonyl groups. A halogen atom, alkoxy group, alkyl group, hydroxyl group, halogeno alkyl group, and halogeno alkoxy group are particularly preferable as a substituent(s). Examples of halogeno alkanoyl groups include a trichloroacetyl group and trifluoroacetyl group. Examples of alkylsulfonyl groups include a methanesulfonyl group and ethanesulfonyl group. Arylsulfonyl groups include a benzenesulfonyl group and p-toluenesulfonyl group. Heteroarylsulfonyl groups include a pyridylsulfonyl group. Halogeno alkylsulfonyl groups include a trifluoromethanesulfonyl group. Alkyloxycarbonyl groups include a methoxycarbonyl group, ethoxycarbonyl group, and tert-butoxycarbonyl group, and aryl-substituted alkoxycarbonyl groups include a benzyloxycarbonyl group and 9-fluorenylmethoxycarbonyl group.

Examples of substituted carbamoyl groups include a methylcarbamoyl group, phenylcarbamoyl group, and substituted phenylcarbamoyl group. A halogen atom, alkoxy group, alkyl group, hydroxyl group, halogeno alkyl group, and halogeno alkoxy group are particularly preferable as a substituent(s). Examples of substituted thiocarbamoyl groups include a methylthiocarbamoyl group, phenylthiocarbamoyl group, and substituted phenylthiocarbamoyl group. A halogen atom, alkoxy group, alkyl group, hydroxyl group, halogeno alkyl group, and halogeno alkoxy group are particularly preferable as a substituent(s). A substituted amino group in the present specification indicates a monosubstituted or disubstituted amino group. The substituents thereof include a C₁₋₆ alkyl group, C₁₋₆ alkyl group substituted with an aryl group(s), C₁₋₆ alkyl group substituted with a heteroaryl group(s), C₁₋₆ alkanoyl group, aroyl group, halogeno C₁₋₆ alkanoyl group, C₁₋₆ alkylsulfonyl group, arylsulfonyl group, heteroarylsulfonyl group, halogeno alkylsulfonyl group, C₁₋₆ alkyloxycarbonyl group, aryl-substituted C₁₋₆ alkyloxycarbonyl groups, substituted or unsubstituted carbamoyl group, and substituted or unsubstituted thiocarbamoyl group. Ammonium groups include a trialkylammonium group, for example.

Since phenylalanine compounds of the formula (1) of the present invention include asymmetric carbons, optical isomers are possible. The compounds of the present invention include such optical isomers and L-form thereof is preferable.

As for the compounds of which a diastereomer(s) exists, the diastereomer(s) and diastereomer mixture(s) are included in the compounds of the present invention. Further, since phenylalanine compounds of the formula (1) of the present invention include mobile hydrogen atoms, various tautomers are possible, and the compounds of the present invention include such tautomers. A carboxyl group in the compounds of the present invention may be substituted by a suitable group(s) that are converted into a carboxyl group *in vivo.* Examples of such groups include a C₁₋₆ alkoxycarbonyl group.

When the compounds of the formula (1) of the present invention can form salts thereof, such salts should be pharmaceutically acceptable ones. For example, to acidic groups such as a carboxyl group in the formula, examples of the salts include salts with alkali metals (such as sodium, potassium, and ammonium); salts with alkaline earth metals (such as calcium and magnesium); aluminum salts; zinc salts; salts with organic amines (such as triethylamine, ethanolamine, morpholine, piperidine, and dicyclohexylamine); and salts with basic amino acids (such as arginine and lysine).

To basic groups in case that a basic group(s) exists in the formula, examples of the salts include salts with inorganic acids (such as hydrochloric acid, sulfuric acid, and phosphoric acid); salts with organic carboxylic acids (such as acetic acid, citric acid, benzoic acid, maleic acid, fumaric acid, tartaric acid, and succinic acid); and salts with organic sulfonic acids (such as methanesulfonic acid and p-toluenesulfonic acid). Regarding methods for forming salts, salts can be obtained by mixing the compounds of the formula (1) with a necessary acid or base at a suitable quantitative ratio in a solvent(s) or dispersant(s); or by conducting cation exchange or anion exchange with other form of salts.

The compounds of the present invention also include solvates such as hydrates and alcohol adducts of the compounds of the formula (1).

The compound (I) or pharmaceutically acceptable salts thereof can be produced by the method described in WO02-16329 (Patent Literature 1). Examples of the Compound (I) include Examples 1 to 213 described in WO02-16329 (Patent Literature 1).

The phenylalanine compound of the formula (1) is preferably a compound wherein R1 represents a methyl group or ethyl group; and R2, R3, and R4 represent a hydrogen atom, halogen atom, hydroxyl group, substituted C₁₋₆ alkyl group, substituted C₂₋₆ alkenyl group, substituted C₂₋₈ alkynyl group, heteroaryl group, hydroxy C₁₋₆ alkyl group, amino group substituted with a C₁₋₆ alkyl group(s), or carbamoyl group substituted with a C₁₋₆ alkyl group(s), wherein substituents in the substituted C₁₋₆ alkyl group, the substituted C₂₋₆ alkenyl group and the substituted C₂₋₈ alkynyl group include an amino group, amino group substituted with a C₁₋₆ alkyl group(s), carboxyl group, C₁₋₆ alkoxycarbonyl group, cyano group, C₁₋₆ alkylthio group, and C₁₋₆ alkylsulfonyl group.

The compound (I) or pharmaceutically acceptable salts thereof are preferably Examples 1, 108, 162, 169, 122, 66, 99, 75, 147, 148, 202, 201, 196, 193, 198 or 197 described in WO02-16329 (EP-A-1,288,205) (Patent Literature 1). They are shown as follows.

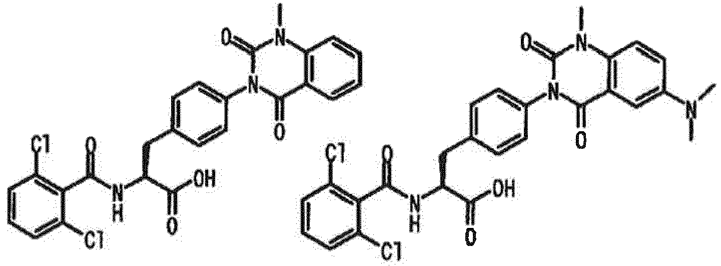

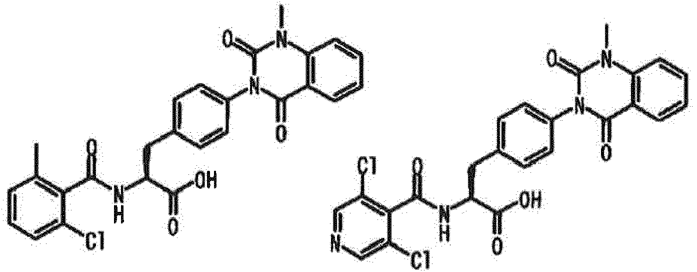

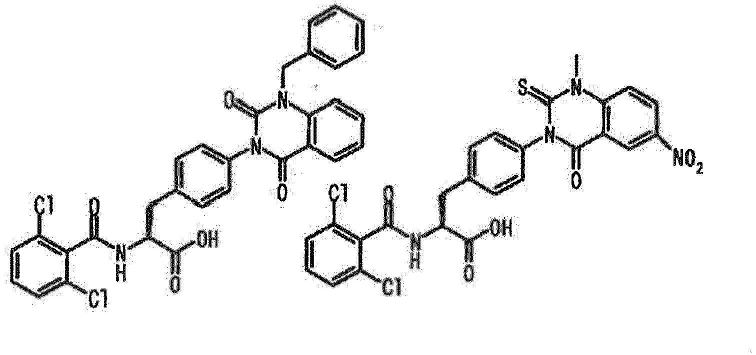

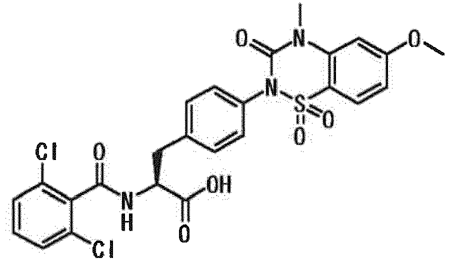

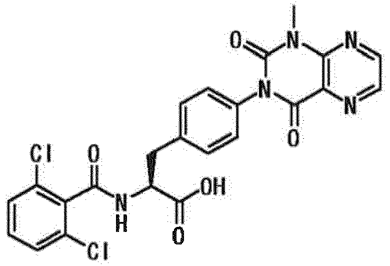

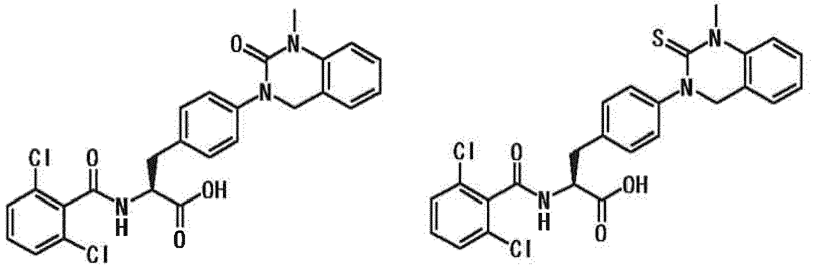

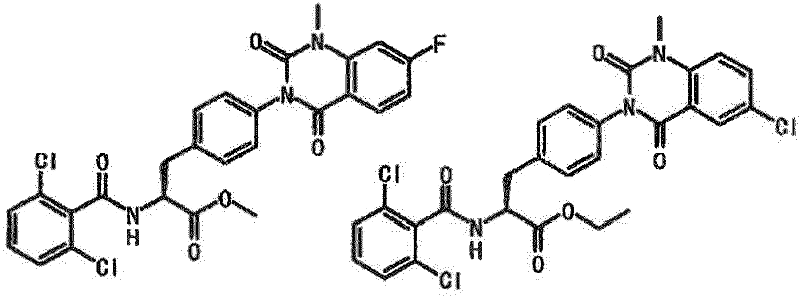

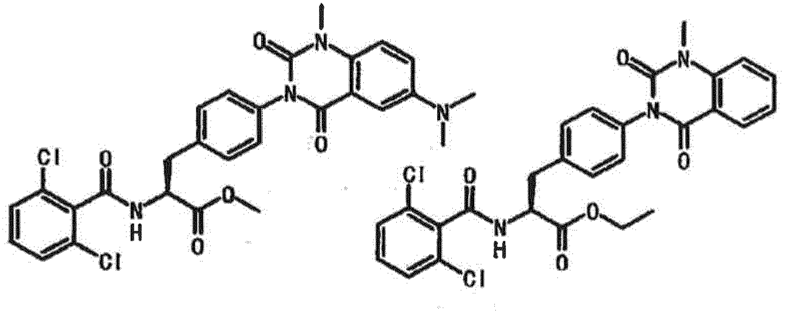

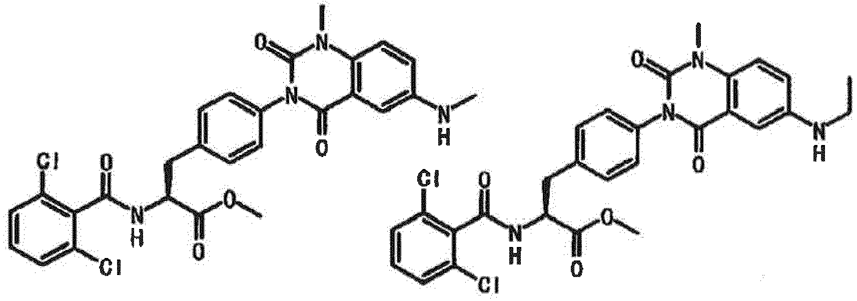

Most preferable one is Example 196 described in WO02-16329 (Patent Literature 1). The present compound (hereinafter referred to as a compound (A)) is shown as follows.

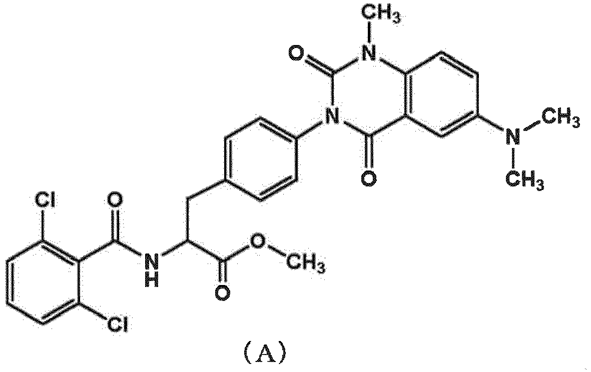

A "sustained-release oral administration preparation" of the present invention also includes "delayed release type oral administration preparations" in addition to so-called "sustained-release type oral administration preparations."

The above "sustained-release type oral administration preparations" are usually preparations which are planned to gradually release drugs from the preparations within digestive tracts regardless of in the stomach or the intestines. The present invention includes both a controlled release type, which maintains the constant concentration of drugs in blood plasma and continuance of the drugs' action is expected; and a prolonged release type, which cannot maintain the constant concentration of drugs in blood plasma, but continuance of the drugs' action can be expected.

Besides, the above "delayed release type oral administration preparations" are preparations which are planned to delay the transport time of preparations from the stomach to the small bowel or to release drugs from the preparations in the bowel parts rather than the stomach. The present invention includes enteric coated preparations.

"Coating preparations" in the present invention are those of which core part containing the drugs (the compound (I) or the compound (A), or pharmaceutically acceptable salts thereof) is coated with a coating material(s) (a water-soluble coating material(s) or a water-insoluble coating material(s)).

In coating preparations using "a water-soluble coating material(s)," the drugs are exposed and released outside because the coating material(s) gradually dissolves in digestive tracts or decays. Other than that, the drugs are exposed and released outside because the coating material(s) dissolves in bowels or disintegrates when being transported to bowels, not in the stomach. The present preparations can control the release of drugs from the preparations by rates of the dissolution or disintegration of "a water-soluble coating material(s)." Preparations coated with a water-soluble coating material(s) include, for example, enteric coated tablets (such as tablets and pills), enteric granules, enteric fine granules, enteric capsules, and suspensions or emulsions containing drugs coated with enteric materials.

For example, in case of coating preparation treated with a water-soluble coating, the dissolution rate of the compound (1) in the preparation depends on pH and is preferably less than 20% to the dissolution rate for 1 hour in pH1.2 (The Japanese Pharmacopoeia Fourteenth Edition, No. 1 solution of the disintegration test).

A "water-soluble coating material(s)" is a pharmaceutically acceptable substance that dissolves in gastrointestinal solutions, and includes the following compounds.

### 1. Acrylic acid derivatives

methacrylic acid copolymer L, methacrylic acid copolymer S, methacrylic acid copolymer LD, aminoalkyl methacrylate copolymer E, and the like.

### 2. Cellulose derivatives

hydroxypropylmethylcellulose phthalate, hydroxypropylmethylcellulose acetate succinate, carboxymethylethylcellulose, cellulose acetate phthalate, hydroxyethylcellulose, hydroxypropylcellulose, hydroxypropylmethylcellulose, methylcellulose, methylhydroxyethylcellulose, opadry, carmellose calcium, carmellose sodium, and the like.

### 3. Vinyl derivatives

polyvinylacetal diethylaminoacetate, polyvinylpyrrolidone, polyvinyl alcohol, and the like.

### 4. Natural polymers and sugars

shellac, gelatin, agar, gum Arabic, pullulan, keratin, and the like.

One, two or more of these "water-soluble coating materials" can be used.

They are preferably methacrylic acid copolymer L, methacrylic acid copolymer, at least S, methacrylic acid copolymer LD, hydroxypropylmethylcellulose phthalate, hydroxypropylmethylcellulose acetate succinate, carboxymethyl- ethylcellulose, cellulose acetate phthalate, polyvinylacetal diethylaminoacetate, shellac, gelatin, and agar.

They are more particularly preferably methacrylic acid copolymer L, methacrylic acid copolymer S, methacrylic acid copolymer LD, hydroxypropylmethylcellulose phthalate, and hydroxypropylmethylcellulose acetate succinate.

On the other hand, in coating preparations using "a water-insoluble coating material(s)," the drugs are gradually released outside via the coating material(s). The present preparations can control the release of drugs by the interruption of the "water-insoluble coating material(s)." Preparations coated with a water-insoluble coating material(s) include, for example, sustained-release coated tablets (such as tablets and pills), sustained-release granules, sustained-release fine granules, sustained-release capsules, and suspensions or emulsions containing drugs coated with sustained-release materials.

For example, in case of coating preparation treated with a water-insoluble coating material(s), the dissolution rate of the compound (1) from the preparation 1 hour later is preferably less than 50% in pH6.8 (1/4 diluted McIlvaine buffer containing 5% (W/V) sodium dodecyl sulfate).

The above dissolution rate can be calculated by conducting the dissolution test in accordance with the Japanese Pharmacopoeia Fourteenth Edition No. 2 in conditions of: paddle rotation speed: 50rpm, test solution quantity: 900mL, test solution: 1/4 diluted McIlvaien buffer (pH6.8) + 5% (W/V) sodium dodecyl sulfate, test solution temperature: 37 ±0.5°C.

A "water-insoluble coating material(s)" is a pharmaceutically acceptable substance that hardly dissolves in gastrointestinal solutions, and includes the following compounds.

### 1. Cellulose derivatives

ethylcellulose, acetylcellulose, cellulose acetate, cellulose propionate, butyl cellulose, and the like.

### 2. Acrylic acid derivatives

aminoalkyl methacrylate copolymer RS, aminoalkyl methacrylate copolymer RL, ethyl acrylate methyl methacrylate copolymer/emulsion, and the like.

### 3. Vinyl derivatives

polyvinylacetate, polyvinylbutylate, and the like.

### 4. Esters

glyceride of a fatty acid of plant and animal origin and mixtures thereof, hydrogenated oils of glyceride of plant and animal origin (such as hydrogenated castor oil and hydrogenated canola oil), glyceride of fatty acids such as an oleic acid, a linoleic acid, a linolenic acid and a linosinic acid, and mixtures thereof, cholesteryl palmitate, palmitates of plant sterol, and the like.

### 5. Fatty acids

lauric acid, tridecanoic acid, myristic acid, pentadecanoic acid, palmitic acid, margaric acid, stearic acid, nanodecanoic acid, arachidic acid, behenic acid, lignoceric acid, cerotic acid, montanic acid, oleic acid, linoleic acid, linolenic acid, capric acid, caproic acid, and the like.

### 6. Alcohols

pentadecanol, hexadecanol, heptadecanol, octadecanol, nanodecanol, eicosanol, wool alcohols, cholesterol, and the like.

### 7. Surfactants

polyoxyethylene hydrogenated castor oil 10, 30, sucrose fatty acid ester, sorbitan tri-fatty acid ester, sorbitan sesqui-fatty acid ester, sorbitan mono-fatty acid ester, glyceryl mono-fatty acid ester, and the like.

One, two or more of these "water-insoluble coating materials" can be used.

They are preferably ethylcellulose, acetylcellulose, cellulose acetate, cellulose propionate, aminoalkyl methacrylate copolymer RS, aminoalkyl methacrylate copolymer RL, ethyl acrylate methyl methacrylate copolymer/emulsion, polyvinylacetate, polyvinylbutylate, glyceride of a fatty acid of plant and animal origin and mixtures thereof, hydrogenated oils of glyceride of plant and animal origin (such as hydrogenated castor oil and hydrogenated canola oil), and glyceride of fatty acids such as an oleic acid, linoleic acid, linolenic acid and linosinic acid, and mixtures thereof.

The coating quantity of water-soluble coating materials or water-insoluble coating materials is 1 to 50 wt% of the solid content coverage of the preparation in tablets, preferably 2 to 30 wt% thereof and further preferably 5 to 30 wt%; and 1 to 100 wt% thereof in granules, preferably 2 to 50 wt% thereof and further preferably 5 to 50 wt%. In this connection, the solid content coverage indicates the solid content quantity (part by weight) of a coating agent coating a tablet to 100 parts by weight of crude tablets before coating (same as below).

The coated preparations of the present invention may have, for example, "the compound (I) or the compound (A), or pharmaceutically acceptable salts thereof', or the mixture of "the compound (I) or the compound (A), or pharmaceutically acceptable salts thereof" and a pharmaceutically acceptable additive(s) as the quick release part in the coated core part. Particularly, the core part is preferably formed by the solid dispersions wherein the compound (I), the compound (A) or pharmaceutically acceptable salts thereof are dispersed in water-soluble polymeric substances in amorphous state.

Such solid dispersions can be prepared using the water-soluble polymeric substances by applying either steps of (i) dissolving or dispersing the above compound (I) or pharmaceutically acceptable salts thereof in an organic solvent(s) together with a water-soluble polymeric substance(s), and then removing the organic solvent(s); (ii) dissolving or dispersing the above compound (I) or pharmaceutically acceptable salts thereof in a water-soluble polymeric substance(s) under heating, and then cooling the mixture; (iii) dissolving or dispersing the above compound (I) or pharmaceutically acceptable salts thereof in a water-soluble polymeric substance(s) under heating and under pressure, and then cooling the mixture; and (iv) mixing the above compound (I) or pharmaceutically acceptable salts thereof together with a water-soluble polymeric substance(s), and then crushing the mixture.

The coating part of the preparations of the present invention may contain "the compound (I) or the compound (A), or pharmaceutically acceptable salts thereof' themselves.

The preparations of the present invention can be produced in accordance with the ordinary methods by spray-drying to the core part containing the drugs an organic solvent(s) such as ethanol or the water-soluble coating material(s) that is dissolved or dispersed in water, using fluid bed granulation coating equipment, centrifugal fluid coating equipment, or vented drying coating equipment.

When preparing the sustained-release oral administration preparations of the present invention, additives can be added, if necessary, such as excipients like sugars, e.g. lactose, sucrose, glucose, hydrogenated maltose, mannitol, sorbitol, xylitol and trehalose, starches and derivatives thereof, e.g. partially α starch, dextrin, pullulan, corn starch and potato starch, celluloses, e.g. crystalline cellulose, microcrystalline cellulose, crystalline cellulose / carmellose sodium and hydroxypropyl cellulose, magnesium aluminometasilicate, silicon dioxide, light anhydrous silicic acid, and amino acids; coloring agents; flavoring agents, e.g. sucrose, aspartame, mannitol, dextran, saccharin, menthol, citric acid, tartaric acid, malic acid, ascorbic acid, sweet hydrangea leaves, fennel, ethanol, fructose, xylitol, glycyrrhizinic acid, purified sucrose, L-glutamine and cyclodextrin; disintegrating agents, e.g. hydroxypropyl cellulose, low-substituted hydroxypropyl cellulose, croscarmellose sodium, α starch, methylcellulose, sodium alginate, sodium carboxymethyl starch, carmellose calcium, carmellose sodium, crystalline cellulose and crystalline cellulose / carmellose sodium; and surfactants, e.g. sodium lauryl sulfate, polysolvate 80, sucrose fatty acid ester, polyoxyl 40 stearate, polyoxyethylene 60 hydrogenated caster oil, sorbitan monostearate and sorbitan monopalmitate.

The present preparations can be either dosage forms of tablets, granules, capsules, microcapsules, pills, dispersants, or subtle granules, and particularly preferably tablets, granules, and capsules.

When granulating the present preparations, they are preferably granulated by agitation granulation, fluid bed granulation, extruding granulation, and spray-drying with agitation granulators, fluid bed granulators, extruding granulators, and spray-dryers and the like.

The administration amount is determined by the intended treatment effect, treatment period, age, and body weight, and the dosage of "the compound (I) or the compound (A), or pharmaceutically acceptable salts thereof' is 1 *µ*g to 5 *µ*g by oral administration per a day for adults.

The present invention includes the coating preparations that can release the compound (I) or pharmaceutically acceptable salts thereof in the lower part of the small bowel.

The oral administration preparations of the present invention have excellent sustained-release property and continuous effects thereof. They are useful since they can decrease number of dosing the drugs and, as a result, improve QOL (quality of life) or compliance of patients.

Examples will further illustrate the sustained-release oral administration preparations of the present invention. They only explain the present invention and do not particularly limit the invention. Comparative examples indicate solid dispersion preparations that are not sustained-released. Meanwhile, in the following description, the compound (A) is Example 196 in WO02/16329 (Patent Literature 1). The following diluting tablets represent the tablets that are prepared by using inactive excipients such as granulated lactose.

### (Comparative Example 1) Tablets (not sustained-released)

792.0g of methanol was put into 216.0g of methylcellulose (SM4, Shin-Etsu Chemical Co., Ltd.) to moisten a whole methylcellulose. 3169.0g of dichloromethane was added thereto, stirred and dissolved. Then, 144.0g of the compound (A) was further added thereto, stirred and dissolved. Thus prepared solution was used as a spray solution mentioned below. 56.0g of partially α starch (PCS PC-10, Asahi Kasei Corporation), 44.8g of croscarmellose sodium (Ac-Di-Sol, Asahi Kasei Corporation), 84.0g of crystalline cellulose (Avicel PH102, Asahi Kasei Corporation) and 22.4g of mannitol (Parteck M200, MERCK) were put into the container of a fluid bed granulator (FLO-1, Freund Corporation), mixed and dried at intake temperature of 90°C. Then, 4230g of the spray solution was sprayed in the conditions of the spray air pressure of 0.15Mpa; and spray speed of 30g/min. to conduct the fluid bed granulation. After the completion of spraying, the mixture was dried by the fluid bed granulator to obtain granules. 0.5% magnesium stearate was added to the obtained granules and tableted to obtain uncoated tablets. The obtained uncoated tablets were film coated with hydroxypropylmethylcellulose (TC-5R, Shin-Etsu Chemical Co., Ltd.) to obtain a solid dispersion preparation (not sustained-released).

### (Comparative Example 2) Granules (not sustained-released)

792.9g of methanol was put into 216.0g of methylcellulose (SM4, Shin-Etsu Chemical Co., Ltd.) to moisten a whole methylcellulose. 3207.5g of dichloromethane was added thereto, stirred and dissolved. Then, 144.0g of the compound (A) was further added thereto, stirred and dissolved. Thus prepared solution was used as a spray solution mentioned below. 337.5g of purified sucrose spheres (Nonpareil 103, Freund Corporation) was put into the bath of a fluid bed granulator (FLO-1, Freund Corporation), mixed and dried at intake temperature of 90°C. Then, 2730g of the spray solution was sprayed in the conditions of the spray air pressure of 0.15Mpa; and spray speed of 30g/min. to conduct the fluid bed granulation. After the completion of spraying, the mixture was dried by the fluid bed granulator. Further, the mixture was sifted to 500 *µ*m to 850 *µ*m to obtain granules.

### (Comparative Example 3) Tablets (Not sustained released)

825.0g of methanol was put into 225.1g of methylcellulose (SM4, Shin-Etsu Chemical Co., Ltd.) to moisten a whole methylcellulose. 3300g of dichloromethane was added thereto, stirred and dissolved. Then, 150.1g of the compound (A) was further added thereto, stirred and dissolved. Thus prepared solution was used as a spray solution mentioned below. 56.0g of partially *α* starch (PCS PC-10, Asahi Kasei Corporation), 44.8g of croscarmellose sodium (Ac-Di-Sol, Asahi Kasei Corporation), 84.0g of crystalline cellulose (Avicel PH102, Asahi Kasei Corporation) and 22.4g of mannitol (Mannit P, TOWA CHEMICAL INDUSTRY CO., LTD.) were put into the bath of a fluid bed granulator (FLO-1, Freund Corporation), mixed and dried at intake temperature of 90°C. Then, 4230g of the spray solution was sprayed in the conditions of the spray air pressure of 0.15Mpa; and spray speed of 30g/min. to conduct the fluid bed granulation. After the completion of spraying, the mixture was dried by the fluid bed granulator to obtain granules. 0.5% magnesium stearate was added to the obtained granules and tableted to obtain uncoated tablets. The obtained uncoated tablets were film coated with hydroxypropylmethylcellulose (TC-5R, Shin-Etsu Chemical Co., Ltd.) to obtain a solid dispersion preparation (not sustained released).

### (Example 1) A coating preparation wherein methacrylic acid copolymer L was used as a water-soluble coating material

3.5g of Macrogol 6000 (NOF Corporation) and 94.0g of purified water were added to a solution wherein 35.0g of methacrylic acid/ethyl acrylate copolymer (Eudragit L100-55, Higuchi Shokai Co., Ltd. (Rohm GmbH)) was dissolved in 350.1g of ethanol, stirred and dissolved. Then, 17.5g of talc (Matsumura Sangyo Co., Ltd.) was added thereto, stirred and dispersed to prepare a coating solution. 60g of the uncoated tablets obtained in Comparative Example 1 and 240g of diluted tablets were film coated using 150.0g of the above coating solution with a coating machine (Highcoater HCT-MINI, Freund Corporation) to prepare Eudragit L100-55 coating tablets (solid part coverage of 10%).

### (Example 2) A coating preparation wherein methacrylic acid copolymer S was used as a water-soluble coating material

3.5g of Macrogol 6000 (NOF Corporation) and 94.0g of purified water were added to a solution wherein 35.0g of methacrylic acid/methyl acrylate copolymer (Eudragit S100, Higuchi Shokai Co., Ltd. (Rohm GmbH)) was dissolved in 350.0g of ethanol, stirred and dissolved. Then, 17.5g of talc (Matsumura Sangyo Co., Ltd.) was added thereto, stirred and dispersed to prepare a coating solution. 60g of the uncoated tablets obtained in Comparative Example 1 and 240g of diluted tablets were film coated using 295.7g of the above coating solution with a coating machine (Highcoater HCT-MINI, Freund Corporation) to prepare Eudragit S100 coating tablets (solid part coverage of 10%).

### (Example 3) A coating preparation wherein hydroxypropylmethylcellulose phthalate was used as a water-soluble coating material

3.5g of Macrogol 6000 (NOF Corporation) was added to a solution wherein 35.0g of hydroxypropylmethylcellulose phthalate (HP-55, Shin-Etsu Chemical Co., Ltd.) was dissolved in the mixed solution of 350.0g of ethanol and 91.5g of purified water, stirred and dissolved. Then, 5.0g of talc (Matsumura Sangyo Co., Ltd.) was added thereto, stirred and dispersed to prepare a coating solution. 60g of the uncoated tablets obtained in Comparative Example 1 and 240g of diluted tablets were film coated using 332.4g of the above coating solution with a coating machine (Highcoater HCT-MINI, Freund Corporation) to prepare HP-55 coating tablets (solid part coverage of 10%).

### (Example 4) A coating preparation wherein ethylcellulose was used as a water-insoluble coating material

3.0g of triethyl citrate (Culter Food Science Co., Ltd.) and 75.0g of purified water were added to a solution wherein 30.0g of ethylcellulose (Ethocel STD-7P, Dow Chem.) was dissolved in 425.2g of ethanol, stirred and dissolved. Then, 15.0g of talc (Matsumura Sangyo Co., Ltd.) was added thereto, stirred and dispersed to prepare a coating solution. 60g of the uncoated tablets obtained in Comparative Example 1 and 240g of diluted tablets were film coated using 295.0g of the above coating solution with a coating machine (Highcoater HCT-MINI, Freund Corporation) to prepare Etcell STD-7P coating tablets (solid part coverage of 10%).

### (Example 5) A coating preparation wherein methacrylic acid copolymer L was used as a water-soluble coating material

3.5g of Macrogol 6000 (NOF Corporation) and 94.0g of purified water were added to a solution wherein 35.0g of methacrylic acid/ethyl acrylate copolymer (Eudragit L100-55, Higuchi Shokai Co., Ltd. (Rohm GmbH)) was dissolved in 350.0g of ethanol, stirred and dissolved. Then, 17.5g of talc (Matsumura Sangyo Co., Ltd.) was added thereto, stirred and dispersed to prepare a coating solution. 60g of the uncoated tablets obtained in Comparative Example 1 and 240g of diluted tablets were film coated using 321.4g of the above coating solution with a coating machine (Highcoater HCT-MINI, Freund Corporation) to prepare Eudragit L100-55 coating tablets (solid part coverage of 15%).

### (Example 6) A coating preparation wherein methacrylic acid copolymer S was used as a water-soluble coating material

3.5g of Macrogol 6000 (NOF Corporation) and 94.0g of purified water were added to a solution wherein 35.0g of methacrylic acid/methyl acrylate copolymer (Eudragit S100, Higuchi Shokai Co., Ltd. (Rohm GmbH)) was dissolved in 350.0g of ethanol, stirred and dissolved. Then, 17.5g of talc (Matsumura Sangyo Co., Ltd.) was added thereto, stirred and dispersed to prepare a coating solution. 30g of the Eudragit S100 coating tablets (solid part coverage of 10%) obtained in Example 2 and 270g of diluted tablets were film coated using 85.0g of the above coating solution with a coating apparatus (Highcoater HCT-MINI, Freund Corporation) to prepare Eudragit S100 coating tablets (solid part coverage of 15%).

### (Example 7) A coating preparation wherein hydroxypropylmethylcellulose phthalate was used as a water-soluble coating material

3.5g of Macrogol 6000 (NOF Corporation) was added to a solution wherein 35.0g of hydroxypropylmethylcellulose phthalate (HP-55, Shin-Etsu Chemical Co., Ltd.) was dissolved in the mixed solution of 350.0g of ethanol and 91.5g of purified water, stirred and dissolved. Then, 5.0g of talc (Matsumura Sangyo Co., Ltd.) was added thereto, stirred and dispersed to prepare a coating solution. 30g of the HP-55 coating tablets (solid part coverage of 10%) obtained in Example 3 and 240g of diluted tablets were film coated using 104.9g of the above coating solution with a coating machine (Highcoater HCT-MINI, Freund Corporation) to prepare HP-55 coating tablets (solid part coverage of 15%).

### (Example 8) A coating preparation wherein ethylcellulose was used as a water-insoluble coating material

3.0g of triethyl citrate (Culter Food Science Co., Ltd.) and 75.0g of purified water were added to a solution wherein 30.0g of ethylcellulose (Ethocel STD-7P, Dow Chem.) was dissolved in 425.0g of ethanol, stirred and dissolved. Then, 17.5g of talc (Matsumura Sangyo Co., Ltd.) was added thereto, stirred and dispersed to prepare a coating solution. 30g of the Etocel STD-7P coating tablets (solid part coverage of 10%) obtained in Example 4 and 240g of diluted tablets were film coated using 140.0g of the above coating solution with a coating apparatus (Highcoater HCT-MINI, Freund Corporation) to prepare Etocel STD-7P coating tablets (solid part coverage of 15%).

### (Example 9) A coating granulated preparation wherein methacrylic acid copolymer L was used as a water-soluble coating material

21.0g of Macrogol 6000 (NOF Corporation) and 572.7g of purified water were added to a solution wherein 210.3g of methacrylic acid/ethyl acrylate copolymer (Eudragit L100-55, Higuchi Shokai Co., Ltd. (Rohm GmbH)) was dissolved in 2100.6g of ethanol, stirred and dissolved. Then, 105.0g of talc (Matsumura Sangyo Co., Ltd.) was added thereto, stirred and dispersed to prepare a coating solution. 120g of the spherical granules obtained in Comparative Example 2 was coated using 1287.1g of the above coating solution with a fluid bed granulator (FLO-1, Freund Corporation) to prepare Eudragit L100-55 coating granules (solid part coverage of 61%).

Test examples will further illustrate effect of sustained-release preparations of the present invention.

### (Test Example 1) A coating preparation

The dissolution test was conducted on the coating preparations of the present invention obtained in Examples 1 to 3 and 5 to 7 and the preparation obtained in Comparative Example 1. In the dissolution test, the acid resistance of 40mg of the compound (A) was evaluated from the dissolution rate two hours later in accordance with the Japanese Pharmacopoeia Fourteenth Edition No. 2 in conditions of: paddle rotation speed: 50rpm, test solution: solution 1.2 of USP (the United States Pharmacopoeia) 24, test solution temperature: 37±0.5°C.

### (Test Example 2) A coating preparation

The dissolution test was conducted on the coating preparations of the present invention obtained in Examples 1 to 4 and the preparation obtained in Comparative Example 1. The dissolution test was conducted to 40mg of the compound (A) in accordance with the Japanese Pharmacopoeia Fourteenth Edition No. 2 in conditions of: paddle rotation speed: 50rpm, test solution: 1/4 diluted McIlvaine buffer (pH6.8) + 5% (W/V) sodium dodecyl sulfate, test solution temperature: 37±0.5°C.

Similarly, the dissolution test was conducted on the coating preparations of the present invention obtained in Examples 5 to 8 and the preparation obtained in Comparative Example 1.

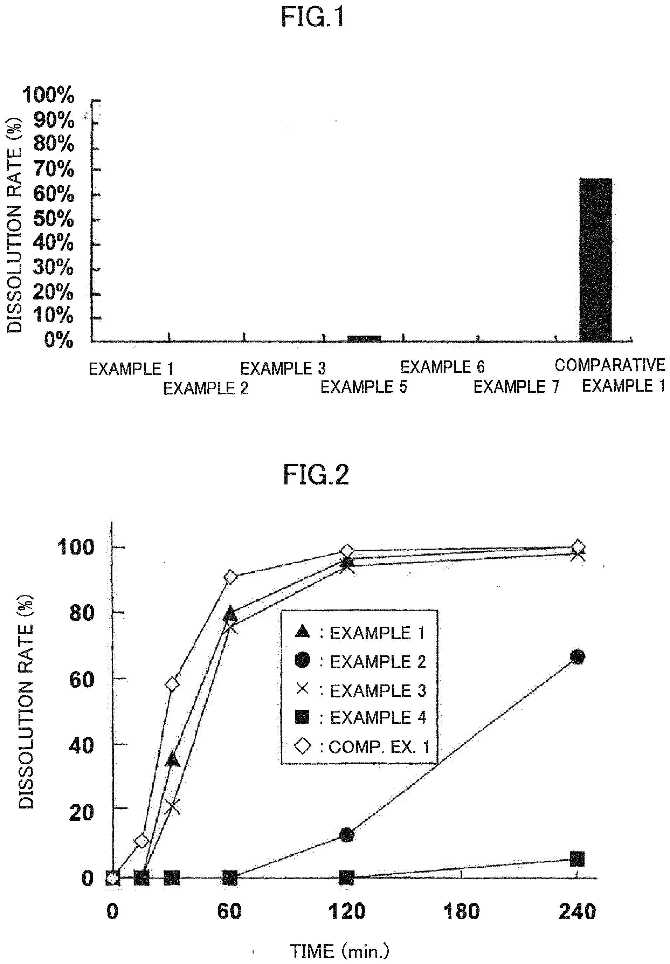

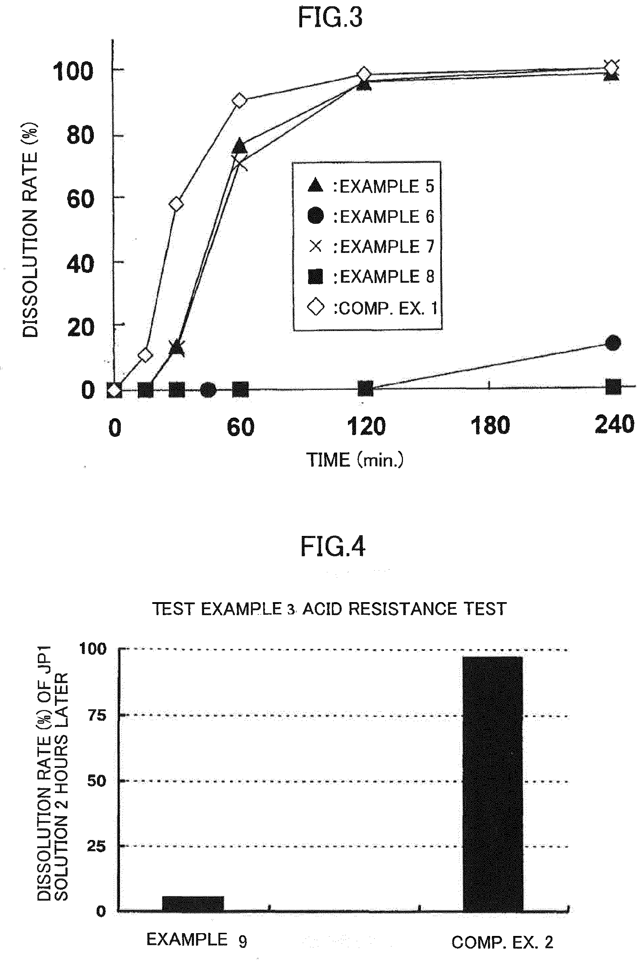

As indicated in Figs. 1 to 3, the release of the compound (A) from the preparation of Comparative Example 1 was rapid, while the release of the compound (A) from the preparations of the present invention obtained in Examples 1 to 4 and 5 to 8 was gradual. Particularly, the acid resistance was seen in the preparations of the present invention using a water-soluble coating material, obtained in Examples 1 to 3 and 5 to 7.

Thus, it has been clarified that the release of the compound (A) from the coating preparations of the present invention has been sufficiently controlled.

### (Test Example 3) Acid resistance test

The dissolution test was conducted on the coating preparations of the present invention obtained in Example 9 and the preparation obtained in Comparative Example 2. In the dissolution test, the acid resistance of 40mg of the compound (A) was evaluated from the dissolution rate two hours later in accordance with the Japanese Pharmacopoeia Fourteenth Edition No. 2 in conditions of: paddle rotation speed: 50rpm, test solution: JP 1.2 solution, test solution temperature: 37±0.5°C.

### (Test Example 4) Pharmacokinetic parameters (Tmax)

40mg equivalents of the preparations of the present invention obtained Examples 3 and 9 and Comparative Examples 1 to 3 were orally administered to male beagle dogs under fasting. The samples of the blood plasma were taken before the administration and 0.25, 0.5, 1, 2, 4, 6, 8, 12 and 24 hours after starting the administration and the maximum blood concentrations (Tmax) were shown in Table 1 as pharmacokinetic parameters. In this connection, Tmax indicates the time to reach the maximum blood concentration.

**Table 1 Pharmacokinetic parameters (Tmax)**

| | Tmax(hr) |
|---|---|
| Comparative Exam. 2 | 0.5 |
| Comparative Exam. 3 | 1.3 |
| Example 3 | 3.7 |
| Example 9 | 1.2 |

### (Test Example 5) The dissolution test at pH6.8

The dissolution test was conducted on the preparation of the present invention (enteric coated preparations) obtained in Example 9. The dissolution test was conducted to 40mg of the compound (A) in accordance with the Japanese Pharmacopoeia Fourteenth Edition No. 2 in conditions of: paddle rotation speed: 50rpm, test solution: 1/4 diluted McIlvaine buffer (pH6.8) + 5% SDS, test solution temperature: 37±0.5°C. The good release of the drugs was seen at pH6.8.

The present invention provides sustained-release oral administration preparations of compounds of formula (1) and pharmaceutically acceptable salts thereof. The sustained-release oral administration preparations of the present invention have an α 4 integrin inhibiting activity and are useful as therapeutic agents or preventive agents for inflammatory diseases in which α 4 integrin-depending adhesion process participates in the pathology, rheumatoid arthritis, inflammatory bowel diseases, systemic erythematodes, multiple sclerosis, Sjogren's syndrome, asthma, psoriasis, allergy, diabetes, cardiovascular diseases, arterial sclerosis, restenosis, tumor proliferation, tumor metastasis and transplantation rejection.

## Claims

1. A sustained-release oral administration preparation having a core part containing an active ingredient, and a coating material for controlling release of the active ingredient from the core part, wherein the coating material is a water-soluble or a water-insoluble coating material, and the active ingredient is a phenylalanine compound of the following formula (1) or a pharmaceutically acceptable salt thereof: 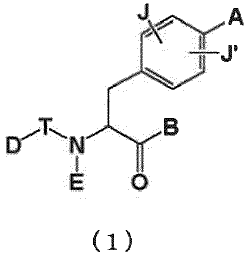 wherein A represents the following formula (2): 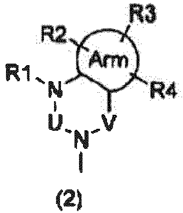
wherein Arm is a cyclic alkyl group or an aromatic ring having 0, 1, 2, 3, or 4 hetero atoms selected from the group consisting of an oxygen atom, sulfur atom and nitrogen atom; U, and V, represent C(=O), S(=O)₂, C(-R5)(-R6), C(=C(R5)(R6)), C(=S), S(=O), P(=O)(-OH), or P(-H)(=O);
wherein R1, R2, R3, R4, R5, and R6 may be same or different from each other and represent a hydrogen atom, halogen atom, hydroxyl group, C₁₋₆ alkyl group, substituted C₁₋₆ alkyl group, C₂₋₆ alkenyl group, substituted C₂₋₆ alkenyl group, C₂₋₈ alkynyl group, substituted C₂₋₈ alkynyl group, cycloalkyl group which may have a hetero atom(s) in the ring, aryl group, heteroaryl group, C₁₋₆ alkoxy group, C₁₋₆ alkylthio group, C₁₋₆ alkoxy group and C₁₋₆ alkylthio group substituted with a cycloalkyl group(s) which may have a hetero atom(s) in the ring, C₁₋₆ alkoxy group and C₁₋₆ alkylthio group substituted with an aryl group(s), C₁₋₆ alkoxy group and C₁₋₆ alkylthio group substituted with a heteroaryl group(s), cycloalkyloxy group which may have a hetero atom(s) in the ring, aryloxy group, heteroaryloxy group, hydroxy C₁₋₆ alkyl group, hydroxy C₂₋₆ alkenyl group, hydroxy C₁₋₆ alkoxy group, halogeno C₁₋₆ alkyl group, halogeno C₁₋₆ alkoxy group, halogeno C₁₋₆ alkylthio group, halogeno C₂₋₆ alkenyl group, nitro group, cyano group, substituted or unsubstituted amino group, carboxyl group, C₁₋₆ alkyloxy carbonyl group, substituted or unsubstituted carbamoyl group, C₁₋₆ alkanoyl group, aroyl group, C₁₋₆ alkylsulfonyl group, substituted or unsubstituted sulfamoyl group, or ammonium group; R5 and R6 may bond together to form a ring, and in the ring, one or two oxygen atoms, nitrogen atoms or sulfur atoms may be included if necessary;
B represents a hydroxyl group, C₁₋₆ alkoxy group, or hydroxylamino group; E represents a hydrogen atom, C₁₋₆ alkyl group, C₂₋₆ alkenyl group, C₂₋₈ alkynyl group, C₁₋₆ alkyl group substituted with a cycloalkyl group(s) which may have a hetero atom(s) in the ring, C₁₋₆ alkyl group substituted with an aryl group(s), or C₁₋₆ alkyl group substituted with a heteroaryl group(s);
D represents a C₁₋₆ alkyl group, C₂₋₆ alkenyl group, C₂₋₈ alkynyl group, cycloalkyl group which may have a hetero atom(s) in the ring, aryl group, heteroaryl group, C₁₋₆ alkyl group substituted with a cycloalkyl group(s) which may have a hetero atom(s) in the ring, C₁₋₆ alkyl group substituted with an aryl group(s), or C₁₋₆ alkyl group substituted with a heteroaryl group(s), C₁₋₆ alkoxy group, C₁₋₆ alkoxy group substituted with a cycloalkyl group(s) which may have a hetero atom(s) in the ring, C₁₋₆ alkoxy group substituted with an aryl group(s), C₁₋₆ alkoxy group substituted with a heteroaryl group(s), a cycloalkyloxy group which may have a hetero atom(s) in the ring, aryloxy group, heteroaryloxy group, hydroxy C₁₋₆ alkyl group, hydroxy C₂₋₆ alkenyl group, hydroxy C₁₋₆ alkoxy group, halogeno C₁₋₆ alkyl group, halogeno C₁₋₆ alkoxy group, halogeno C₂₋₆ alkenyl group, nitro group, cyano group, substituted or unsubstituted amino group, carboxyl group, C₁₋₆ alkyloxy carbonyl group, substituted or unsubstituted carbamoyl group, C₁₋₆ alkanoyl group, aroyl group, C₁₋₆ alkylthio group, C₁₋₆ alkylsulfonyl group, or substituted or unsubstituted sulfamoyl group; E and D may bond together to form a ring, and in the ring, one or two oxygen atoms, nitrogen atoms or sulfur atoms may be included if necessary;
T represents an interatomic bond, C(=O), C(=S), S(=O), S(=O)₂, N(H)-C(=O), or N(H)-C(=S); and
J and J' may be same or different from each other and represents a hydrogen atom, halogen atom, C₁₋₆ alkyl group, C₁₋₆ alkyloxy group, or nitro group.

2. The sustained-release oral administration preparation according to claim 1 wherein the core part is formed of a solid dispersion in which the active ingredient is dispersed in a water-soluble polymeric substance in amorphous state.

3. The sustained-release oral administration preparation according to claim 1 or 2, wherein the core part containing the active ingredient is coated with a water-soluble coating material.

4. The sustained-release oral administration preparation according to claim 3, wherein the water-soluble coating material is at least one of acrylic acid derivatives, cellulose derivatives, vinyl derivatives, and natural polymers and sugars.

5. The sustained-release oral administration preparation according to claim 3, wherein the water-soluble coating material is at least one of methacrylic acid copolymer L, methacrylic acid copolymer S, methacrylic acid copolymer LD, hydroxypropylmethylcellulose phthalate, hydroxypropylmethylcellulose acetate succinate, carboxymethylethylcellulose, cellulose acetate phthalate, polyvinylacetal diethylaminoacetate, shellac, gelatin, and agar.

6. The sustained-release oral administration preparation according to claim 3, wherein the water-soluble coating material is at least one of methacrylic acid copolymer L, methacrylic acid copolymer S, methacrylic acid copolymer LD, hydroxypropylmethylcellulose phthalate, and hydroxypropylmethylcellulose acetate succinate.

7. The sustained-release oral administration preparation according to any one of claims 3 to 6, wherein the solid content coverage is 1 to 50 wt% in tablets, and 1 to 100 wt% in granules.

8. The sustained-release oral administration preparation according to any one of claims 3 to 6, wherein the solid content coverage is 2 to 30 wt% in tablets, and 2 to 50 wt% in granules.

9. The sustained-release oral administration preparation according to claim 1 or 2, wherein the core part containing the active ingredient is coated with a water-insoluble coating material.

10. The sustained-release oral administration preparation according to claim 9, wherein the water-insoluble coating material is at least one of cellulose derivatives, acrylic acid derivatives, vinyl derivatives, esters, fatty acids, alcohols and surfactants.

11. The sustained-release oral administration preparation according to claim 9, wherein the water-insoluble coating material is at least one of ethylcellulose, acetylcellulose, cellulose acetate, cellulose propionate, aminoalkyl methacrylate copolymer RS, aminoalkyl methacrylate copolymer RL, ethyl acrylate methyl methacrylate copolymer/emulsion, polyvinylacetate, polyvinylbutylate, glyceride of a fatty acid of plant or animal origin and mixtures thereof, hydrogenated oils of glyceride of plant or animal origin, and glycerides of fatty acids.

12. The sustained release oral administration preparation of claim 11 wherein the water-insoluble coating material comprises at least one glyceride of fatty acid selected from oleic acid, linoleic acid, linolenic acid and linosinic acid, and mixtures thereof.

13. The sustained-release oral administration preparation according to any one of claims 9 to 12, wherein the solid content coverage is 1 to 50 wt% in tablets, and 1 to 100 wt% in granules.

14. The sustained-release oral administration preparation according to any one of claims 9 to 12, wherein the solid content coverage is 2 to 30 wt% in tablets, and 2 to 50 wt% in granules.

15. The sustained-release oral administration preparation according to any one of claims 1 to 14, wherein the phenylalanine compound of the formula (1) or pharmaceutically acceptable salt thereof is a compound or pharmaceutically acceptable salt thereof wherein, in the formula (1), R1 represents a methyl group or ethyl group; and R2, R3, and R4 represent a hydrogen atom, halogen atom, hydroxyl group, substituted C₁₋₆ alkyl group, substituted C₂₋₆ alkenyl group, substituted C₂₋₈ alkynyl group, heteroaryl group, hydroxy C₁₋₆ alkyl group, amino group substituted with a C₁₋₆ alkyl group(s), or carbamoyl group substituted with a C₁₋₆ alkyl group(s), wherein substituents in the substituted C₁₋₆ alkyl group, the substituted C₂₋₆ alkenyl group and the substituted C₂₋₈ alkynyl group include an amino group, amino group substituted with a C₁₋₆ alkyl group(s), carboxyl group, C₁₋₆ alkoxycarbonyl group, cyano group, C₁₋₆ alkylthio group, and C₁₋₆ alkylsulfonyl group.

16. The sustained-release oral administration preparation according to any one of claims 1 to 14, wherein the phenylalanine compound of the formula (1) or pharmaceutically acceptable salts thereof is compound (A) or pharmaceutically acceptable salt thereof.

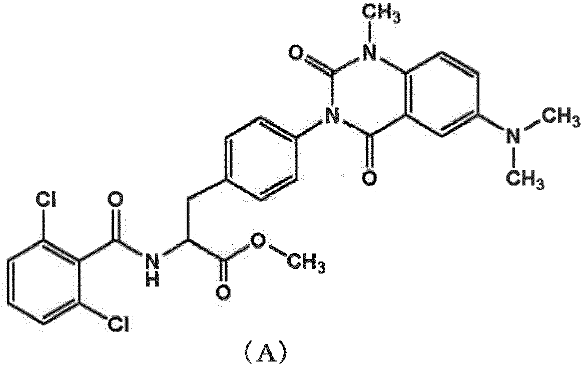

## Patentansprüche

1. Zubereitung mit verzögerter Freisetzung für die orale Verabreichung, die einen Kernteil, der einen aktiven Bestandteil enthält, und ein Beschichtungsmaterial zum Kontrollieren der Freisetzung des aktiven Bestandteils aus dem Kernteil aufweist, wobei das Beschichtungsmaterial ein wasserlösliches oder wasserunlösliches Beschichtungsmaterial ist und der aktive Bestandteil eine Phenylalaninverbindung der Formel (1) oder ein pharmazeutisch geeignetes Salz davon ist: 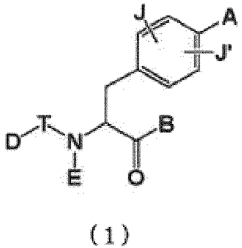 worin A die folgende Formel (2) darstellt: 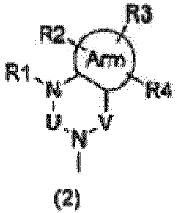
worin Arm eine zyklische Alkylgruppe oder ein aromatischer Ring mit 0, 1, 2, 3 oder 4 Heteroatomen ist, das aus der Gruppe ausgewählt sind, die aus einem Sauerstoffatom, Schwefelatom und Stickstoffatom besteht; U und V C(=O), S(=O)₂, C(-R5)(-R6), C(=C(R5)(R6)), C(=S), P(=O)(·OH) oder P(·H)(=O) darstellen;
worin R1, R2, R3, R4, R5 und R6 gleich oder verschieden voneinander sein können und ein Wasserstoffatom, Halogenatom, eine Hydroxygruppe, eine C₁₋₆-Alkylgruppe, eine substituierte C₁₋₆-Alkylgruppe, eine C₂₋₆-Alkinylgruppe, eine substituierte C₂₋₆-Alkenylgruppe, eine C₂₋₈-Alkinylgruppe, eine substituierte C₂₋₈-Alkinylgruppe, eine Cycloalkylgruppe, die ein oder mehrere Heteroatome im Ring aufweisen kann, eine Arylgruppe, eine Heteroarylgruppe, eine C₁₋₆-Alkoxygruppe, C₁₋₆-Alkylthiogruppe, C₁₋₆-Alkoxygruppe und eine C₁₋₆-Alkylthiogruppe, die mit einer oder mehreren Cycloalkylgruppen, die ein oder mehrere Heteroatome im Ring aufweisen können, substituiert ist, eine C₁₋₆-Alkoxygruppe und eine C₁₋₆-Alkylthiogruppe, die mit einer mehreren Arylgruppen substituiert ist, eine C₁₋₆-Alkylgruppe und eine C₁₋₆-Alkylthiogruppe, die mit einer oder mehreren Heteroarylgruppen substituiert ist, eine Cycloalkyloxygruppe, die ein oder mehrere Heteroatome im Ring aufweisen kann, eine Aryloxygruppe, Heteroaryloxygruppe, Hydroxy-C₁₋₆-alkylgruppe, Hydroxy-C₂₋₆-alkenylgruppe, Hydroxy-C₁₋₆-alkoxygruppe, Halogen-C₁₋₆-alkylgruppe, Halogen-C₁₋₆-alkoxygruppe, Halogen-C₁₋₆-alkylthiogruppe, Halogen-C₂₋₆-alkenylgruppe, Nitrogruppe, Cyangruppe, eine substituierte oder unsubstituierte Amingruppe, eine Carboxygruppe, C₁₋₆-Alkyloxycarbonylgruppe, eine substituiert oder unsubstituierte Carbamoylgruppe, eine C₁₋₆-Alkanoylgruppe, Aroylgruppe, C₁₋₆-Alkylsulfonylgruppe, eine substituiert oder unsubstituierte Sulfamoylgruppe oder eine Ammoniumgruppe darstellen; R5 und R6 unter Bildung eines Rings aneinander gebunden sein können, wobei in dem Ring ein oder zwei Sauerstoffatome, Stickstoffatome oder Schwefelatome bei Bedarf enthalten sein können;
B eine Hydroxygruppe, C₁₋₆-Alkoxygruppe oder eine Hydroxylamingruppe darstellt;
E ein Wasserstoffatom, eine C₁₋₆-Alkylgruppe, eine C₂₋₆-Alkenylgruppe, C₂₋₈-Alkinylgruppe, C₁₋₆-Alkylgruppe, die mit einer oder mehreren Cycloalkylgruppen, die ein oder mehrere Heteroatome im Ring aufweisen können, substituiert ist, eine C₁₋₆-Alkylgruppe, die mit einer oder mehreren Arylgruppen substituiert ist, oder eine C₁₋₆-Alkylgruppe darstellt, die mit einer mehreren Heteroarylgruppen substituiert ist;
D eine C₁₋₆-Alkylgruppe, C₂₋₆-Alkenylgruppe, C₂₋₈-Alkinylgruppe, eine Cycloalkylgruppe, die ein oder mehrere Heteroatome im Ring aufweisen kann, eine Arylgruppe, Heteroarylgruppe, C₁₋₆-Alkylgruppe, die mit einer oder mehreren Cycloalkylgruppen, die ein oder mehrere Heteroatome im Ring aufweisen können, substituiert ist, eine C₁₋₆-Alkylgruppe, die mit einer oder mehreren Arylgruppen substituiert ist, oder eine C₁₋₆-Alkylgruppe, die mit einer oder mehreren Heteroarylgruppen substituiert ist, eine C₁₋₆-Alkoxygruppe, C₁₋₆-Alkoxygruppe, die mit einer oder mehreren Cycloalkylgruppen, die ein oder mehrere Heteroatome im Ring aufweisen können, substituiert ist, eine C₁₋₆-Alkoxygruppe, die mit einer oder mehreren Arylgruppen substituiert ist, eine C₁₋₆-Alkoxygruppe, die mit einer oder mehreren Heteroarylgruppen subsituiert ist, eine Cycloalkyloxygruppe, die ein oder mehrere Heteroatome im Ring aufweisen kann, eine Aryloxygruppe, eine Heteroaryloxygruppe, eine Hydroxy-C₁-₆-alkylgruppe, eine Hydroxy-C₂₋₆-alkenylgruppe, eine Hydroxy-C₁₋₆-alkoxygruppe, eine Halogen-C₁₋₆-alkylgruppe, Halogen-C₁₋₆-alkoxygruppe, Halogen-C₂₋₆-alkenylgruppe, Nitrogruppe, Cyangruppe, substituierte oder unsubstituierte Amingruppe, Carboxygruppe, C₁₋₆-Alkyloxycarbonylgruppe, eine substituiert oder unsubstituierte Carbamoylgruppe, eine C₁₋₆-Alkanoylgruppe, Aroylgruppe, C₁₋₆-Alkylthiogruppe, C₁₋₆-Alkylsulfonylgruppe oder eine substituierte oder unsubstituierte Sulfamoylgruppe darstellt;
E und D unter Bildung eines Rings aneinander gebunden sein können, wobei in dem Ring ein oder zwei Sauerstoffatome, Stickstoffatome und Schwefelatome bei Bedarf enthalten sein können;
T eine Zwischenatombindung, C(=O), C(=S), S(=O), S(=O)₂, N(H) ·C(=O) oder N(H) ·C(=S) darstellt; und
J und J' gleich oder verschieden voneinander sein können und ein Wasserstoffatom, Halogenatom, eine C₁₋₆-Alkylgruppe, C₁₋₆-Alkyloxygruppe oder Nitrogruppe darstellen.

2. Zubereitung mit verzögerter Freisetzung für die orale Verabreichung nach Anspruch 1, wobei der Kernteil aus einer festen Dispersion gebildet ist, worin der aktive Bestandteil in einer wasserlöslichen Polymersubstanz in einem amorphen Zustand dispergiert ist.

3. Zubereitung mit verzögerter Freisetzung für die orale Verabreichung nach Anspruch 1 oder 2, wobei der den aktiven Bestandteil enthaltende Kernteil mit einem wasserlöslichen Beschichtungsmaterial überzogen ist.

4. Zubereitung mit verzögerter Freisetzung für die orale Verabreichung nach Anspruch 3, wobei das wasserlösliche Beschichtungsmaterial mindestens ein unter Acrylsäurederivaten, Zellulosederivaten, Vinylderivaten und natürlichen Polymeren und Zuckern ausgewähltes Material ist.

5. Zubereitung mit verzögerter Freisetzung für die orale Verabreichung nach Anspruch 3, wobei das wasserlösliche Beschichtungsmaterial mindestens ein unter Methacrylsäurecopolymer L, Methacrylsäurecopolymer S, Methacrylsäurecopolymer LD, Hydroxypropylmethylzellulosephthalat, Hydroxypropylmethylzelluloseacetatsuccinat, Carboxymethylethylzellulose, Zelluloseacetatphthalat, Polyvinylacetaldiethylaminoacetat, Shellac, Gelatine und Agar ausgewähltes Material ist.

6. Zubereitung mit verzögerter Freisetzung für die orale Verabreichung nach Anspruch 3, wobei das wasserlösliche Beschichtungsmaterial mindestens ein unter Methacrylsäurecopolymer L, Methacrylsäurecopolymer S, Methacrylsäurecopolymer LD, Hydroxypropylmethylzellulosephthalat und Hydroxypropylmethylzelluloseacetatsuccinat ausgewähltes Material ist.

7. Zubereitung mit verzögerter Freisetzung für die orale Verabreichung nach einem der Ansprüche 3 bis 6, wobei der Feststoffgehaltsanteil 1 bis 50 Gew.-% in Tabletten und 1 bis 100 Gew.-% in Granulaten ist.

8. Zubereitung mit verzögerter Freisetzung für die orale Verabreichung nach einem der Ansprüche 3 bis 6, wobei der Feststoffgehaltsanteil 2 bis 30 Gew.-% in Tabletten und 2 bis 50 Gew.-% in Granulaten ist.

9. Zubereitung mit verzögerter Freisetzung für die orale Verabreichung nach Anspruch 1 oder 2, wobei der den aktiven Bestandteil enthaltende Kernteil mit einem wasserunlöslichen Beschichtungsmaterial überzogen ist.

10. Zubereitung mit verzögerter Freisetzung für die orale Verabreichung nach Anspruch 9, wobei das wasserunlösliche Beschichtungsmaterial mindestens ein unter Zellulosederivaten, Acrylsäurederivaten, Vinylderivaten, Estern, Fettsäuren, Alkohol und oberflächenaktiven Mitteln ausgewähltes Material ist.

11. Zubereitung mit verzögerter Freisetzung für die orale Verabreichung nach Anspruch 9, wobei das wasserunlösliche Beschichtungsmaterial mindestens ein unter Etyhlzellulose, Acetylzellulose, Zelluloseacetat, Zellulosepropionat, Aminalkylmethacrylatcopoylmer RS, Aminalkylmethacrylatcopolymer RL, Ethylacrylatmethylmethacrylatcopolymer-Emulsion, Polyvinylacetat, Polyvinylbutylat, Glycerid einer Fettsäure pflanzlichen oder tierischen Ursprungs und Gemischen davon, hydrierten Ölen eines Glycerids pflanzlichen oder tierischen Ursprungs und Glyceriden von Fettsäuren ausgewähltes Material ist.

12. Zubereitung mit verzögerter Freisetzung für die orale Verabreichung nach Anspruch 11, wobei das wasserunlösliche Beschichtungsmaterial mindestens ein Glycerid einer Fettsäure aufweist, die unter Ölnsäue, Linolsäure, Linolensäure und Linosinsäure und Gemischen davon ausgewählt ist.

13. Zubereitung mit verzögerter Freisetzung für die orale Verabreichung nach einem der Ansprüche 9 bis 12, wobei der Feststoffgehaltsanteil 1 bis 50 Gew.-% in Tabletten und 1 bis 100 Gew.-% in Granulaten ist.

14. Zubereitung mit verzögerter Freisetzung für die orale Verabreichung nach einem der Ansprüche 9 bis 12, wobei der Feststoffgehaltsanteil 2 bis 30 Gew.-% in Tabletten und 2 bis 50 Gew.-% in Granulaten ist.

15. Zubereitung mit verzögerter Freisetzung für die orale Verabreichung nach einem der Ansprüche 1 bis 14, wobei die Phenylalaninverbindung der Formel (1) oder ein pharmazeutisch geeignetes Salz davon eine Verbindung oder ein pharmazeutisch geeignetes Salz davon ist, worin in der Formel (1) R1 eine Methylgruppe oder Ethylgruppe darstellt; und R2, R3 und R4 ein Wasserstoffatom, Halogenatom, eine Hydroxygruppe, eine substituierte C₁₋₆-Alkylgruppe, eine substituierte C₂₋₆-Alkenylgruppe, eine substituierte C₂₋₈-Alkenylgruppe, eine Heteroarylgruppe, eine Hydroxy-C₁₋₆-Alkylgruppe, eine mit einer oder mehreren C₁₋₆-Alkylgruppen substituierte Amingruppe oder eine mit einer oder mehreren C₁₋₆-Alkylgruppen substituierte Carbamoylgruppe darstellen, wobei die Substituenten in der substituierten C₁₋₆-Alkylgruppe, der substituierten C₂₋₆-Alkenylgruppe und der substituierten C₂₋₈-Alkinylgruppe eine Amingruppe, eine mit einer oder mehreren C₁₋₆-Alkylgruppen substituierte Amingruppe, eine Carboxgruppe, eine C₁₋₆-Alkoxycarbonylgruppe, eine Cyangruppe, eine C₁₋₆-Alkylthiogruppe und eine C₁₋₆-Alkylsulfonylgruppe umfassen.

16. Zubereitung mit verzögerter Freisetzung für die orale Verabreichung nach einem der Ansprüche 1 bis 14, wobei die Phenylalaninverbindung der Formel 1 oder ein pharmazeutisch geeignetes Salz davon Verbindung (A) oder ein pharmazeutisch geeignetes Salz davon ist.

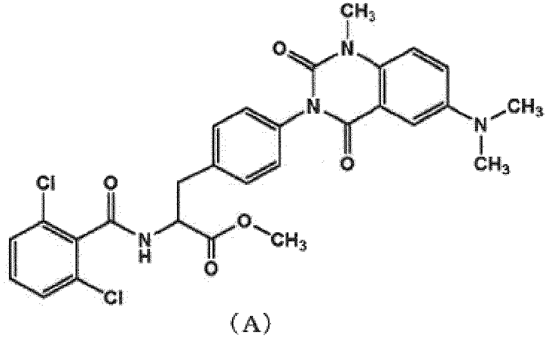

## Revendications

1. Préparation pour une administration orale à libération prolongée présentant une partie de noyau comprenant un ingrédient actif, et un matériau de revêtement pour contrôler la libération de l'ingrédient actif à partir de la partie de noyau, dans laquelle le matériau de revêtement est un matériau de revêtement soluble dans l'eau ou insoluble dans l'eau, et l'ingrédient actif est un composé de phénylalanine de la formule (1) suivante ou un sel pharmaceutiquement acceptable de celui-ci : 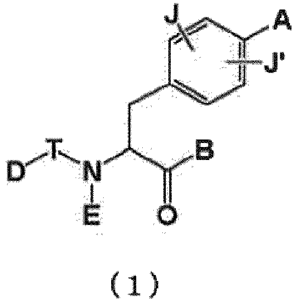 où A représente la formule (2) suivante : 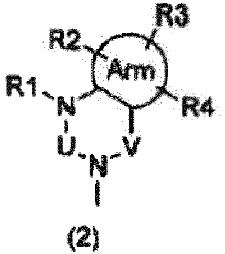
où Arm est un groupe alkyle cyclique ou un noyau aromatique ayant 0, 1, 2, 3, ou 4 hétéroatomes choisis dans le groupe constitué d'un atome d'oxygène, d'un atome de soufre et d'un atome d'azote ; U, et V, représentent C(=O), S(=O)₂, C(-R5)(-R6), C(=C(R5)(R6)), C(=S), S(=O), P(=O)(-OH), ou P(-H)(=O) ;
où R1, R2, R3, R4, R5, et R6 peuvent être identiques ou différents les uns des autres et représentent un atome d'hydrogène, un atome d'halogène, un groupe hydroxyle, un groupe alkyle en C₁₋₆, un groupe alkyle en C₁₋₆ substitué, un groupe alcényle en C₂₋₆, un groupe alcényle en C₂₋₆ substitué, un groupe alcynyle en C₂₋₈, un groupe alcynyle en C₂₋₈ substitué, un groupe cycloalkyle qui peut présenter un(des) hétéroatome(s) dans le noyau, un groupe aryle, un groupe hétéroaryle, un groupe alcoxy en C₁₋₆, un groupe alkylthio en C₁₋₆, un groupe alcoxy en C₁₋₆ et un groupe alkylthio en C₁₋₆ substitué avec un(des) groupe(s) cycloalkyle qui peu(ven)t présenter un(des) hétéroatome(s) dans le noyau, un groupe alcoxy en C₁₋₆ et un groupe alkylthio en C₁₋₆ substitué avec un(des) groupe(s) aryle, un groupe alcoxy en C₁₋₆ et un groupe alkylthio en C₁₋₆ substitué avec un(des) groupe(s) hétéroaryle, un groupe cycloalkyloxy qui peut présenter un(des) hétéroatome(s) dans le noyau, un groupe aryloxy, un groupe hétéroaryloxy, un groupe hydroxyalkyle en C₁₋₆, un groupe hydroxyalcényle en C₂₋₆, un groupe hydroxyalcoxy en C₁₋₆, un groupe halogénoalkyle en C₁₋₆, un groupe halogénoalcoxy en C₁₋₆, un groupe halogénoalkylthio en C₁₋₆, un groupe halogénoalcényle en C₂₋₆, un groupe nitro, un groupe cyano, un groupe amino substitué ou non substitué, un groupe carboxyle, un groupe alkyloxycarbonyle en C₁₋₆, un groupe carbamoyle substitué ou non substitué, un groupe alcanoyle en C₁₋₆, un groupe aroyle, un groupe alkylsulfonyle en C₁₋₆, un groupe sulfamoyle substitué ou non substitué, ou un groupe ammonium ; R5 et R6 peuvent être liés ensemble pour former un noyau, et dans le noyau, un ou deux atomes d'oxygène, atomes d'azote ou atomes de soufre peuvent être inclus si nécessaire ;
B représente un groupe hydroxyle, un groupe alcoxy en C₁₋₆ ou un groupe hydroxylamino ;
E représente un atome d'hydrogène, un groupe alkyle en C₁₋₆, un groupe alcényle en C₂₋₆, un groupe alcynyle en C₂₋₈, un groupe alkyle en C₁₋₆, substitué avec un(des) groupe(s) cycloalkyle qui peuvent présenter un(des) hétéroatome(s) dans le noyau, un groupe alkyle en C₁₋₆ substitué avec un(des) groupe(s) aryle, ou un groupe alkyle en C₁₋₆ substitué avec un (des) groupe(s) hétéroaryle ;
D représente un groupe alkyle en C₁₋₆, un groupe acényle en C₁₋₆, un groupe alcynyle en C₂₋₈, un groupe cycloalkyle qui peut présenter un(des) hétéroatome(s) dans le noyau, un groupe aryle, un groupe hétéroaryle, un groupe alkyle en C₁₋₆ substitué avec un(des) groupe(s) cycloalkyle qui peu(ven)t présenter un(des) hétéroatome(s) dans le noyau, un groupe alkyle en C₁₋₆ substitué avec un(des) groupe(s) aryle, ou un groupe alkyle en C₁₋₆ substitué avec un(des) groupe(s) hétéroaryle, un groupe alcoxy en C₁₋₆, un groupe alcoxy en C₁₋₆ substitué avec un(des) groupe(s) cycloalkyle qui peuvent présenter un(des) hétéroatome(s) dans le noyau, un groupe alcoxy en C₁₋₆ substitué avec un(des) groupe(s) aryle, un groupe alcoxy en C₁₋₆ substitué avec un(des) groupe(s) hétéroaryle, un groupe cycloalkyloxy qui peut présenter un(des) hétéroatome(s) dans le noyau, un groupe aryloxy, un groupe hétéroaryloxy, un groupe hydroxyalkyle en C₁₋₆, un groupe hydroxyalcényle en C₂₋₆, un groupe hydroxyalcoxy en C₁₋₆, un groupe halogénoalkyle en C₁₋₆, un groupe halogénoalcoxy en C₁₋₆, un groupe halogénoalcényle en C₂₋₆, un groupe nitro, un groupe cyano, un groupe amino substitué ou non substitué, un groupe carboxyle, un groupe alkyloxycarbonyle en C₁₋₆, un groupe carbamoyle substitué ou non substitué, un groupe alcanoyle en C₁₋₆, un groupe aroyle, un groupe alkylthio en C₁₋₆, un groupe alkylsulfonyle en C₁₋₆, ou un groupe sulfamoyle substitué ou non substitué ;
E et D peuvent être liés ensemble pour former un noyau, et dans le noyau, un ou deux atomes d'oxygène, atomes d'azote ou atomes de soufre peuvent être inclus si nécessaire ;
T représente une liaison interatomique, C(=O), C(=S), S(=O), S(=O)₂, N(H)-C(=O), ou N(H)-C(=S) ; et
J et J' peuvent être identiques ou différents l'un de l'autre et représentent un atome d'hydrogène, un atome d'halogène, un groupe alkyle en C₁₋₆, un groupe alkyloxy en C₁₋₆, ou un groupe nitro.

2. Préparation d'administration orale à libération prolongée selon la revendication 1, dans laquelle la partie de noyau est formée d'une dispersion solide dans laquelle l'ingrédient actif est dispersé dans une substance polymère soluble dans l'eau dans un état amorphe.

3. Préparation d'administration orale à libération prolongée selon la revendication 1 ou 2, dans laquelle la partie de noyau contenant l'ingrédient actif est revêtue avec un matériau de revêtement soluble dans l'eau.

4. Préparation d'administration orale à libération prolongée selon la revendication 3, dans laquelle le matériau de revêtement soluble dans l'eau est au moins un de dérivés d'acide acrylique, de dérivés de cellulose, de dérivés vinyliques, et de polymères naturels et de sucres.

5. Préparation d'administration orale à libération prolongée selon la revendication 3, dans laquelle le matériau de revêtement soluble dans l'eau est au moins un d'un copolymère d'acide méthacrylique L, d'un copolymère d'acide méthacrylique S, d'un copolymère d'acide méthacrylique LD, de phtalate d'hydroxypropylméthylcellulose, de succinate acétate d'hydroxypropylméthylcellulose, de carboxyméthyléthylcellulose, de phtalate acétate de cellulose, de diéthylaminoacétate de polyvinylacétal, de shellac, de gélatine et d'agar.

6. Préparation d'administration orale à libération prolongée selon la revendication 3, dans laquelle le matériau de revêtement soluble dans l'eau est au moins un d'un copolymère d'acide méthacrylique L, d'un copolymère d'acide méthacrylique S, d'un copolymère d'acide méthacrylique LD, de phtalate d'hydroxypropylméthylcellulose, et d'acétate succinate d'hydroxypropylméthylcellulose.

7. Préparation d'administration orale à libération prolongée selon l'une quelconque des revendications 3 à 6, dans laquelle la couverture de contenu solide est de 1 à 50 % en masse dans des comprimés, et de 1 à 100 % en masse dans des granulés.

8. Préparation d'administration orale à libération prolongée selon l'une quelconque des revendications 3 à 6, dans laquelle la couverture de contenu solide est de 2 à 30 % en masse dans des comprimés, et de 2 à 50 % en masse dans des granulés.

9. Préparation d'administration orale à libération prolongée selon la revendication 1 ou 2, dans laquelle la partie de noyau comprenant l'ingrédient actif est revêtue avec un matériau de revêtement insoluble dans l'eau.

10. Préparation d'administration orale à libération prolongée selon la revendication 9, dans laquelle le matériau de revêtement insoluble dans l'eau est au moins un de dérivés de cellulose, de dérivés d'acide acrylique, de dérivés vinyliques, d'esters, d'acides gras, d'alcools, et de tensioactifs.

11. Préparation d'administration orale à libération prolongée selon la revendication 9, dans laquelle le matériau de revêtement insoluble dans l'eau est au moins un de l'éthylcellulose, de l'acétylcellulose, de l'acétate de cellulose, du propionate de cellulose, d'un copolymère de méthacrylate d'aminoalkyle RS, d'un copolymère de méthacrylate d'aminoalkyle RL, d'un copolymère/émulsion d'acrylate d'éthyle méthacrylate de méthyle, d'un poly(acétate de vinyle), d'un poly(butyrate de vinyle), d'un glycéride d'un acide gras d'origine végétale ou animale et de mélanges de ceux-ci, d'huiles hydrogénées de glycéride d'origine végétale ou animale, et de glycérides d'acides gras.

12. Préparation d'administration orale à libération prolongée selon la revendication 11, dans laquelle le matériau de revêtement insoluble dans l'eau comprend au moins un glycéride d'acide gras choisi parmi l'acide oléique, l'acide linoléique, l'acide linolénique et l'acide linosinique, et de mélanges de ceux-ci.

13. Préparation d'administration orale à libération prolongée selon l'une quelconque des revendications 9 à 12, dans laquelle la couverture de contenu solide est de 1 à 50 % en masse dans des comprimés, et de 1 à 100 % en masse dans des granulés.

14. Préparation d'administration orale à libération prolongée selon l'une quelconque des revendications 9 à 12, dans laquelle la couverture de contenu solide est de 2 à 30 % en masse dans des comprimés, et de 2 à 50 % en masse dans des granulés.

15. Préparation d'administration orale à libération prolongée selon l'une quelconque des revendications 1 à 14, dans laquelle le composé de phénylalanine de la formule (1) ou sel pharmaceutiquement acceptable de celui-ci est un composé ou sel pharmaceutiquement acceptable de celui-ci où, dans la formule (1), R1 représente un groupe méthyle ou un groupe éthyle ; et R2, R3, et R4 représentent un atome d'hydrogène, un atome d'halogène, un groupe hydroxyle, un groupe alkyle en C₁₋₆ substitué, un groupe alcényle en C₂₋₆ substitué, un groupe alcynyle en C₂₋₈ substitué, un groupe hétéroaryle, un groupe hydroxy(alkyle en C₁₋₆), un groupe amino substitué avec un(des) groupe(s) alkyle en C₁₋₆, ou un groupe carbamoyle substitué avec un(des) groupe(s) alkyle en C₁₋₆, dans laquelle des substituants dans le groupe alkyle en C₁₋₆ substitué, le groupe alcényle en C₂₋₆ substitué et le groupe alcynyle en C₂₋₈ substitué comprennent le groupe amino, un groupe amino substitué avec un(des) groupe(s) alkyle en C₁₋₆, un groupe carboxyle, un groupe alcoxycarbonyle en C₁₋₆, un groupe cyano, un groupe alkylthio en C₁₋₆, et un groupe alkylsulfonyle en C₁₋₆.

16. Préparation d'administration orale à libération prolongée selon l'une quelconque des revendications 1 à 14, dans laquelle le composé de phénylalanine de la formule (1) ou les sels pharmaceutiquement acceptables de celui-ci est le composé (A) ou des sels pharmaceutiquement acceptables de celui-ci.
